(19) Europäisches Patentamt
European Patent Office
Office européen des brevets

(11) **EP 0 561 130 B1**

(12) **EUROPEAN PATENT SPECIFICATION**

(45) Date of publication and mention
of the grant of the patent:
**23.04.1997 Bulletin 1997/17**

(51) Int Cl.$^6$: **C07K 14/63**, **A61K 38/17**

(21) Application number: **93101406.2**

(22) Date of filing: **29.01.1993**

(54) **Stabilized biologically active polypeptide and use thereof**

Stabilisierte biologisch aktive Polypeptide und deren Verwendung

Polipeptides actives stabilisés et leur utilisation

(84) Designated Contracting States:
**BE CH DE FR GB IT LI**

(30) Priority: **30.01.1992 JP 38338/92**

(43) Date of publication of application:
**22.09.1993 Bulletin 1993/38**

(73) Proprietor: **SANWA KAGAKU KENKYUSHO CO., LTD.**
**Nagoya, Aichi-ken (JP)**

(72) Inventors:
• **Kurono, Masayasu**
  **Nagoya, Aichi-ken (JP)**
• **Mitani, Takahiko**
  **Nagoya, Aichi-ken (JP)**
• **Takahashi, Haruo**
  **Nagoya, Aichi-ken (JP)**
• **Ishii, Yoko**
  **Nagoya, Aichi-ken (JP)**
• **Iida, Takafumi**
  **Nagoya, Aichi-ken (JP)**
• **Ikami, Takao**
  **Nagoya, Aichi-ken (JP)**
• **Sawai, Kiichi**
  **Nagoya, Aichi-ken (JP)**

(74) Representative:
**Wächtershäuser, Günter, Prof. Dr.**
**Patentanwalt,**
**Tal 29**
**80331 München (DE)**

(56) References cited:
**EP-A- 0 378 078**

• **PROC. NATL. ACAD. SCI. USA vol. 84, February 1987, WASHINGTON pages 675 - 679 TIM J. AHERN ET AL. 'Control of oligomeric enzyme thermostability by protein engeneering'**
• **EUR. J. BIOCHEM. vol. 171, 1988, NEW YORK pages 715 - 720 MASAZUMI MATSUMURA ET AL. 'Role of tyrosine-80 in the stability of kanamycin nucleotidyltransferase analyzed by side-directed mutagenesis'**

**Description**

The present invention relates to a stabilized polypeptide showing motilin-like biological activities and use thereof. The stabilized polypeptide according to the invention, for instance, a motilin analogue is stable for a long period of time and holds its biological activity in higher level, so that the peptide can be used as a component for medicines such as an effective ingredient in a remedy for gastrointestinal tract disorders.

EP-A-378 078 discloses (Leu$^{13}$Glu$^{19}$Hse$^{23}$)-motilin. This protein is not part of the present application.

M. Matsumara et al., Eur. J. Biochem., Vol. 171, pp. 715-720 (1980) discloses the replacement of Asp at position 80 by Thr, Ser, Ala, Val, Leu, Phe, and Trp of kanamycin nucleotidyltransferase (KNTase). The enzyme was modified by site-directed mutagenesis and a high stability in case of Tyr$^{80}$ was observed.

T.J. Ahern et al., Proc. Natl. Acad. Sci. USA, Vol. 84, pp. 675-679 (1987) teaches the increase of the half-life of yeast triosephosphate isomerase by replacement of Asn$^{14}$ and Asn$^{78}$ by Thr and Ile, respectively.

In recent years, accompanying with a progress of biotechnology, various peptide preparations containing a certain biologically active polypeptides as an effective ingredient have been developed. However, these peptide preparation have disadvantages in that such biologically active polypeptides are, in general, not so stable, not suitable for a long preservation, and have a short half-value period (half life) in vivo when the peptide preparation is administrated. Incidentally, it has been known that the deamidation is apt to occur at a site of asparagine (Asn) residue, when -Asn-Gly- exists in an amino acid sequence encoding the polypeptide.

It has been reported that an insulin can be stabilized by converting asparagine (Asn) residue at C-terminal of A-chain into another amino acid residue such as glycine (Gly) residue [Jap. Pat. No. Hei 1 (A.D. 1989) - 502434(A)].

The main target of the invention lies in stabilization of peptides with biological activities similar to motilin, although the invention is not exclusively limited to so-called "motilin analogues". Therefore, we shall refer to prior arts on motilins.

Motilin is a kind of peptide hormones which has firstly been isolated from mucosa of porcine upper small intestine and determined its amino acid sequence by Brown et al ["Gastroenterology", Vol. 62, pages 401 - 404 (1972) and "Can. J. Biochem.", Vol. 52, pages 7 - 10 (1974)]. The porcine motilin consists of 22 amino acid residues and has a molecular weight of about 2700.

The present inventors have succeeded in an isolation of cloned cDNA encoding a human motilin and determined its structure. Then, it has been made apparent that an amino acid sequence of the human motilin is same with that of the porcine motilin [Jap. Pat. No. Sho. 63 (A.D. 1988) - 276489(A)].

The motilin has been known to as a hormone having various biological activities, especially controlling a hypermotility action of digestive tract during a hungry period. Since no report has been issued on any specific side-effect for the motilin, it has been considered that motilin is efficient for curing and diagnosing gastroenteropathys at the period of post-operation, in lieu of prostaglandin which widely employed now, although it shows a strong side-effect.

It has also been reported that the native motilin had methionine (Met) residue at 13-position of the amino acid sequence therefor, but a polypeptide having an amino acid sequence, wherein the methionine (Met) residue at 13-position was replaced by leucine (Leu) or norleucine (Nle) residue, showed biological activities in same level with the native motilin ["Scad. J. Gastroenterology", Vol. 11, pages 199 - 203 (1976) et al]. Therefore, it has been considered that the methionine residue at 13-position thereof has almost no important effect upon activities of the motilin.

Further, the present inventors have found out that motilin analogues wherein a part of the amino acid residues was substituted by other amino acid residues or added to some amino acid residues showed activities in the same level with or higher than the native motilin, and have made it apparent that a certain rule or law was existed between the modification and activity of the modified polypeptides [Jap. Pat. No. Hei 3 (A.D. 1991) - 218395(A)].

It has been known that the motilins have relatively low stability similar to other known biologically active polypeptides. For preserving the motilins or preparing a medicine with use of the same, therefore, such a measurement as an adjustment of pH and addition of a stabilizer of arginine or the like amino acid, mannitol or the like saccharide, albumin or the like protein has been taken [Jap. Pat. Nos. Hei 3 (A.D. 1991) - 41032(A) and 41033(A) as well as specification for Jap. Pat. Appln. No. Hei 3 (A.D. 1991) - 76202].

As referred to hereinbefore, the biologically active polypeptides, in general, are not so stable and the activity thereof falls down rapidly, because its side chain of structural amino acids tends to change easily, during its preservation period of time.

Then, though the pH control and/or addition of the stabilizer have been done as referred to, it has been considered as difficult to maintain the stability of polypeptide in the level of an isotonic solution and has never been succeeded to steadily maintain the activities for a long period of time.

Further, it has been issued no report on a stable biologically active polypeptide by itself.

Therefore, a principal object of the invention is to provide a stabilized biologically active polypeptide showing motilin-like biological activities which is stable by itself and thus coexistence of a stabilizer is not always required.

An additional but important object of the invention is to provide a stable peptide preparation or medicine which comprises the stabilized biologically active polypeptide showing motilin-like biological activities as an effective ingredient and thus not always require a specific stabilizer.

The present inventors have energetically studied and investigated on stability of various biologically active polypeptides to find out that the site of asparagine (Asn) and aspartic acid (Asp) residue in the polypeptide is not stable, so that its activities reduces for relatively short period of time, when it was reserved under conventional reserving conditions. Then, they thought that a modified polypeptide, wherein Asn and/or Asp residues in its nature polypeptide were replaced by other stable amino acid residues in a extent of giving substantially no influence on its biological activities, would has an improved stability than the native polypeptide, and under such an assumption, synthesized such modified polypeptides to check biological activities thereof to find that the modified polypeptides had same or higher activities than the native or original polypeptides and an excellent stability, the invention has been established.

Therefore, according to the present invention, the principal object can be achieved according to Claim 1 by a stabilized biologically active polypeptide showing motilin-like biological activities, wherein an asparagine (Asn) and aspartic acid (Asp) residues in an amino acid sequence encoding a physiologically active native polypeptide, existing at a position other than at C-terminal of sequence thereof and giving substantially no influence upon expression of activities are replaced to another amino acid residue.

The ground of that in the stabilized polypeptides according to the invention, the asparagine (Asn) and aspartic acid (Asp) residues to be replaced are specified as "those existing in a position other than at C-terminal" lies in that a reaction mechanism on modification of such amino acid residues, which has been elucidated by the inventors and shall referred to hereinafter, can not be applied for, when such amino acid residues lie at C-terminal.

By the way of study for this invention, it was found that in case of polypeptides and more particularly on motilin and its analogue, a modification or mutation is apt to occur at the site of asparagine (Asn) in 19-position and the reaction mechanism on the modification has been elucidated as stated below. Therefore, the inventors have synthesized polypeptides, each having an amino acid sequence similar to that for motilin but the amino acid residue in 19-position is not asparagine (Asn) or aspartic acid (Asp) residue to check biological activities to find that each of the resulting polypeptides has activities equal to or higher than the basic or fundamental polypeptide and shows an improved stability.

The reaction mechanism on modification or mutation can be estimated as follows. Namely, the asparagine residue in 19-position of the polypeptide is cyclic-imidized to form an anhydroaspartic acid residue, and this residue then changes into $\alpha$ - or $\beta$ -aspartic acid residue, by the action of OH ion. This cyclic-imidization also occur, when the amino acid residue in 19-position is aspartic acid (Asp) residue and the 20th amino acid residue is lysine (Lys) one. In the latter case, it has been confirmed that a part of aspartic acid (Asp) residues in 19-position is changed to $\beta$ -structural one.

Therefore, for the purpose of fundamentally improving a stability of the biologically active polypeptides, it is important to inhibit an occurrence of the modification or mutation of the asparagine (Asn) and aspartic acid (Asp) residues in the constitutional amino acid sequence thereof, by replacing the other stable amino acid residue such as glutamine (Gln), glutamic acid (Glu), alanine (Ala) or the like.

The stabilized biologically active polypeptides according to the invention, having activities similar to motilins are shown by the formula of


## Phe-Val-Pro-Ile-Phe-Thr-Tyr-Gly-Glu-Leu-Gln-Arg- X - Gln-
## Glu-Lys-Glu-Arg- Y - Lys-Gly-Gln- Z


wherein X is Leu, Y is Gln or Ala, and Z is OH or homoserine residue (including homoserine residue (including homoserine-lactone residue and represented by "Hse").

Since when the motilins analogue shown by the formula would be synthesized by a fermentation method, linked plural number of the motilin analogue in tandem arrangement between a methionine (Met) residue or a spacer peptide with Met residue at both ends with use of conventional techniques, introduced the resulting polypeptide into an expression vector, transformed Escherichia coli or the like microorganism or animal cell with the recombinant vector, expressed a fused protein of the tandemly arranged motilin analogues, and then treated this fused protein with cyanogen bromide to cleave or separate the same into individual motilin analogues. If the amino acid residue at X-position will Met residue, please note that said treatment with cyanogen bromide causes the cleaving or separation of the fused protein into individual polypeptide fragments at the position of Met and convert the Met residue to homoserine or homoserine-lactone.

The aforesaid additional object of the invention is achieved by a peptide preparation comprising at least one of the stabilized polypeptide shown by said formula, as an effective ingredient.

The preparation, of course, may contain one or more known polypeptide stabilizer selected from the group consisting of an amino acid, saccharide, protein, alcohol, inhibitor to denaturation or decomposition of proteins, and the like. The stabilized peptide preparation shall be administrated to cure mainly gastrointestinal tract disorders.

The present invention will now be further explained in more detail with reference to a Manufacturing Example; Test Examples on stability and pharmacological activity; and another Test Example for analysis on a mutant to be formed during preservation, which shall refer to drawings, wherein

Fig.1 shows an elution pattern showing a result of an ion exchanged HPLC, when [Leu[13]]motilin-Hse solution with relatively low stability was reserved under a severe condition and then mutant modified in its side chain was separated;

Fig.2 shows an illustration showing the sites of asparagine (Asn) - lysine (Lys) residues in 19 - 20 positions of a motilin analogue, and particularly for explaining a reaction mechanism of modification, which is estimated to occur in asparagine residue.

In the followings, the explanation will be given on a motilin analogue, wherein methionine (Met) residue at 13-position of native motilin is modified into leucine (Leu) residue and having Hse residue at 23-position.

Examples (Manufacturing Examples)

Each synthesis of polypeptides was carried out with the peptide synthesizer type 430A manufactured by Applied Biosystems Co. For instance, a polypeptide with following amino acid sequence was prepared with a separately and chemically synthesized Boc-Hse(Bzl)-4-(oxymethyl)phenyl acetic acid and an aminomethyl resin was employed, and homoserine (Hse) was introduced at C-terminal thereof (Constitutional amino acids were prepared in a conventional manner.

```
Phe-Val-Pro-Ile-Phe-Thr-Tyr-Gly-Glu-Leu-Gln-Arg-Leu-Gln-
Glu-Lys-Glu-Arg-Glu-Lys-Gly-Gln-Hse
```

The resulting synthesized polypeptide sample was purified by means of HPLC with a μ-bondasphere C-18 column (19 mm x 15 cm) manufactured by Waters Co., under the following conditions.

Eluate :　　Linear gradient of 15% to 50% acetonitrile in 0.012N hydrochloric acid; 30 min.
Flow rate:　　7.0 ml/min.

Fractions in a main peak part on the HPLC were recovered and lyophilized. and investigated a part thereof with a peptide sequencer manufactured by Applied Biosystems Co. to confirm a fact that the synthesized polypeptide was identified to have an exact amino acid sequence.

Other polypeptides, a part of an amino acid sequence encoding each of them was different, were also synthesized by the peptide synthesizer and purifying the same, in the manner similar to the above.

Test Example 1 (Stability)

Each of motilin analogues produced was dissolved in a phosphate buffer (pH 5.0) to aseptically prepare 0.lmg/ml of motilin analogue solution which was then charged into glass vials to seal the same. The vials were preserved in an incubator held at 60°C to measure a change of activity of the motilin analogue in the vial with the lapse of time, by the method of HPLC. Results are shown in following Table 1, as remained activity under assumption that an activity just before the preservation was 100%. In the samples given in Table 1, the amino acid residue in 19-position for [Leu[13]] motilin-Hse and [Leu[13]]motilin was asparagine (Asn) residue.

Table 1

| Sample | Remained activity (%) | |
|---|---|---|
| | 10th day | After 1 month |
| [Leu[13], Asp[19]]motilin-Hse | 77.6 | 45.6 |
| [Leu[13]]motilin-Hse | 86.9 | 61.8 |
| [Leu[13], Glu[19]]motilin-Hse | 98.5 | 95.3 |
| [Leu[13], Gln[19]]motilin-Hse | 97.6 | 93.8 |
| [Leu[13], Ala[19]]motilin-Hse | 97.2 | 93.5 |
| [Leu[13]]motilin | 88.9 | 46.2 |

EP 0 561 130 B1

Table 1   (continued)

| Sample | Remained activity (%) | |
|---|---|---|
| | 10th day | After 1 month |
| [Leu$^{13}$, Glu$^{19}$]motilin | 98.7 | 96.2 |

As apparently seen from Table 1, the polypeptides are unstable when they have Asn or Asp residue in 19-position thereof, but the polypeptide wherein Asn or Asp residue is replaced of Gln, Glu or Ala residues is extremely excellent in its stability.

Test Example 2 (Measurement of intestinal canal contraction activity)

An intestinal canal contraction activity was measured in accordance with the Magnus method utilizing a rabbit duodenum muscle, on various motilin analogues obtained by the Example, as test samples to check the activities thereof, under assumption of that the chemically synthesized human motilin shows the activity of 100%. Results are shown in following Table 2.

Table 2

| Sample | Specific activity (%) |
|---|---|
| Motilin | 100 |
| [Leu$^{13}$, Asp$^{19}$]motilin-Hse | 95 |
| [Leu$^{13}$]motilin-Hse | 120 |
| [Leu$^{13}$, Glu$^{19}$]motilin-Hse | 130 |
| [Leu$^{13}$, Gln$^{19}$]motilin-Hse | 110 |
| [Leu$^{13}$, Ala$^{19}$]motilin-Hse | 90 |
| [Leu$^{13}$]motilin | 100 |

As apparently seen from the Table 2, the motilin analogues wherein the Asn residue in 19-position of native type human motilin was replaced to other amino acid residue other than Asn was identified to have the intestinal canal contraction activity of same with or higher than the native type human motilin.

Test Example 3 (Analysis on structure of deamidated products resulted during preservation of motilin analogue)

For this test, following motilin analogue ([Leu$^{13}$]motilin-Hse) was employed, which has been said as having a relatively low stability:

```
Phe-Val-Pro-Ile-Phe-Thr-Tyr-Gly-Glu-Leu-Gln-Arg-Leu-Gln-
Glu-Lys-Glu-Arg-Asn-Lys-Gly-Gln-Hse.
```

(1) Separation and analysis by HPLC

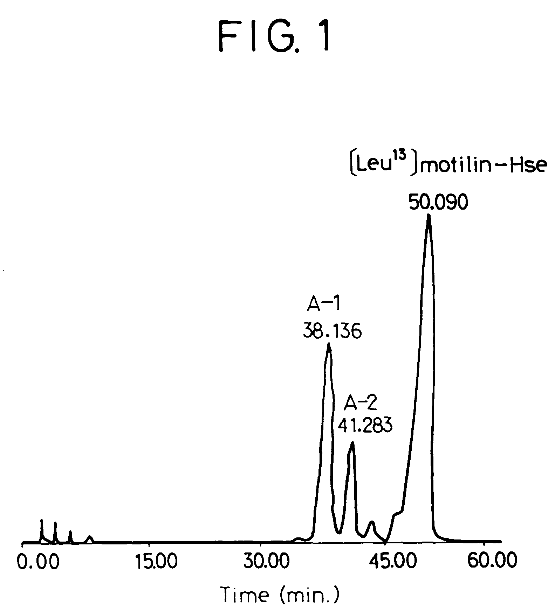

A sample solution was prepared by dissolving the motilin analogue in phosphate buffer (pH 5.0) and preserved in an incubator at 40°C for 60 days. The sample solution was subjected to an ion exchange HPLC under following conditions to separate main deamidated products to be analyzed the same. A separation pattern thereof is shown in Fig. 1.

Condition for Detection : UV ray (230nm),
Column : TSK gel SP2SW (Toso Co.,Ltd.), 5μ m, 4.6 x 250mm,
Temperature of column : 40°C,
Moving phase :

A :        0.02M sodium dihydrogen phosphate (pH 4.6)/acetonitril (9/1) ;
B :        Solution A + 0.2M sodium sulfate ;
A - B :    Linear gradient (30 min.), B (10 min.).

5

Two main deamidated products were separated and named as "A-1" and "A-2" as shown in Fig.1.

(2) Amino acid sequences for A-1 and A-2

Amino acid sequences for A-1 and A-2 were checked with the peptide sequencer 470A manufactured by ABI Co.. The results are shown below. As seen therefrom, the peak for A-1 was only detected until 18-position of Arg. The A-2 was identified to have an amino acid sequence of [Leu13, Asp19]motilin-Hse, which was formed through the conversion of an amino acid residue in 19-position of original [Leu13]motilin-Hse to an aspartic acid (Asp) residue and, but there was found no decrease in yield through the conversion, namely the yield was same level in case of its chemical synthesis.

```
A-1  :  Phe-Val-Pro-Ile-Phe-Thr-Tyr-Gly-Glu-Leu-Gln-Arg-Leu-
        Gln-Glu-Lys-Glu-Arg (not detected hereinafter).


A-2  :  Phe-Val-Pro-Ile-Phe-Thr-Tyr-Gly-Glu-Leu-Gln-Arg-Leu-
        Gln-Glu-Lys-Glu-Arg-Asp-Lys-Gly-Gln-Hse.
```

(3) Amino acid composition for A-1 and A-2

Results of analysis of amino acid compositions for A-1 and A-2 are shown in following Table 3. As apparently seen therefrom, both of A-1 and A-2 had amino acid composition similar to that of the original [Leu13]motilin-Hse. Incidentally, In Table 3, the molar ratio is given as that of Phe was 2.0, and means that an amino acid not described therein was not detected.

In the analysis of amino acid composition, asparagine (Asn) and glutamine (Gln) are detected as aspartic acid (Asp) and glutamic acid (Glu) due to its deamidization. A peak of Hse is also detected for the mutant A-1, and thus it is considered that A-1 has a structure not cleaved between 18- and 19-positions thereof and Asn in 19-position changes into a structure of stopping Edman degradation.

Table 3

| Amino acid | [Leu13]motilin-Hse | A-1 | A-2 |
|:---:|:---:|:---:|:---:|
| Asp | 1.0 | 1.1 | 1.0 |
| Thr | 1.0 | 1.0 | 1.0 |
| Glu | 6.1 | 6.2 | 6.1 |
| Hse | 0.9 | 0.8 | 0.9 |
| Gly | 2.1 | 2.1 | 2.1 |
| Val | 1.1 | 1.1 | 1.0 |
| Ile | 1.0 | 1.0 | 1.0 |
| Leu | 2.0 | 2.0 | 2.0 |
| Tyr | 2.0 | 1.1 | 1.1 |
| Phe | 2.0 | 2.0 | 2.0 |
| Lys | 2.0 | 1.9 | 1.9 |
| Arg | 2.0 | 2.0 | 2.1 |
| Pro | 1.1 | 1.1 | 1.1 |

(4) Mass spectrum

The mass spectrometry was practiced by means of FAB method and with CONCEPT H Type Analyzer manufactured by Claytos Co.. As the result, it has been found that each of A-1 and A-2 was bigger than the original [Leu13] motilin-Hse by one mass.

6

(5) Prediction of structure and reaction mechanism

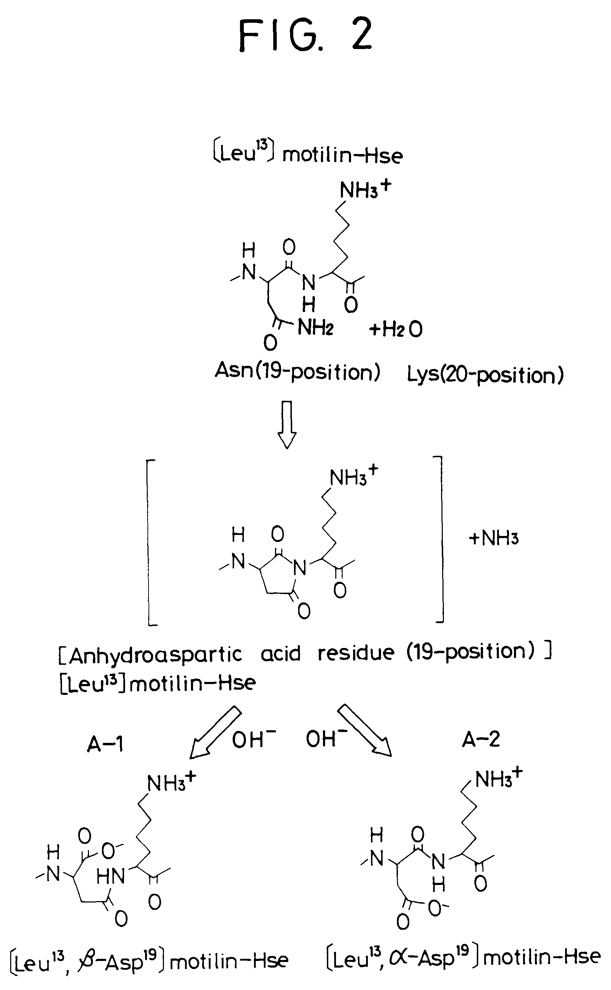

It was considered that the mechanism of deamidation reaction in 19-position of Asn residue may be shown as in Fig. 2, when results of various analysis are taken into consideration. Accordingly, the amino acid residues in 19- and 20-positions of [Leu$^{13}$]motilin-Hse for the sample is Asn-Lys, but the Asn residue in 19-position shall be ring-closed to form anhydroaspartic acid residue as a cyclic imide, and then this cyclic imide was opened by an action of OH ion to be converted into aspartic acid residue. On this occasion, the motilin analogue formed $\alpha$-Asp-Lys linkage took a structure of [Leu$^{13}$, $\alpha$-Asp$^{19}$]motilin-Hse, wherein the Asn residue in 19-position was changed to $\alpha$-Asp residue to afford the mutant A-2. On the other hand, the motilin analogue formed $\beta$-Asp-Lys linkage took a structure of [Leu$^{13}$, $\beta$-Asp$^{19}$]motilin-Hse, whereby the Edman degradation was interrupted to afford the mutant A-1.

Further, the inventors have confirmed such a fact through similar experiments, that the formation of cyclic imide was resulted not only when the 19-position of the motilin analogue was an asparagine (Asn) residue but also when above position was aspartic acid (Asp) residue, and a part thereof took a structure of [Leu$^{13}$, $\beta$-Asp$^{19}$]motilin-Hse, whereby the Edman degradation was interrupted to afford the mutant A-1.

In consequence, it is considered, that the mutation resulted, when the polypeptide was preserved under the usual condition, was caused to form five-membered ring imide compounds through Asn and Asp residues as an intermediate. Therefore, it was found that prevention of forming this intermediate, e.g. cyclic imide compound was important for stabilizing the polypeptide, wherein Asn and Asp residues are contained in the constitutional amino acid sequence, and in other words, it is require to replace Asn and Asp residues by another stable amino acid residue, if the residue of Asn and Asp does not a substantial influence upon the activity of the polypeptide.

Medicine Preparation Examples

(1) Injection

A peptide preparation was prepared by dissolving [Leu$^{13}$, Glu$^{19}$]motilin-Hse obtained by the Example into an acetic acid-sodium acetate buffer (pH 5.0), aseptically charging the resulting solution into ampules, each containing 1 mg as the polypeptide, and sealing the ampules.

The polypeptide solution may be charged into vials, lyophilized and sealed therein to obtain a powdered preparation. The powdered preparation shall be dissolved into saline for injection purpose.

Furthermore, a pharmaceutically acceptable substance selected from group consisting of a saccharide, alcohol, amino acid and protein may be composed into the injectional preparation, as a secondary stabilizer.

(2) Nasal drop

A nasal drop was prepared by dissolving 5mg of [Leu$^{13}$, Gln$^{19}$]motilin-Hse obtained by the Example and 50mg of saponin, into 5ml of purified water. The nasal drop is administrated to apply onto or spray into nasal cavity.

(3) Tablet

With following components, tablets were prepared in accordance with a conventional method.

| | |
|---|---|
| [Leu$^{13}$, Glu$^{19}$]motilin-Hse | 10 (mg) |
| Sodium lauryl sulfate | 20 |
| Carboxymethylcellulose (Ca) | 7 |
| Crystalline cellulose | 2 |
| Magnesium stearate | 7 |
| Lactose | Remainder |
| | 200 mg/tablet |

**Claims**

1. A stabilized polypeptide showing motilin-like biological activities, which has an amino acid sequence of

```
Phe-Val-Pro-Ile-Phe-Thr-Tyr-Gly-Glu-Leu-Gln-Arg-Leu-Gln-
Glu-Lys-Glu-Arg- Y -Lys-Gly-Gln- Z
```

wherein Y is Gln or Ala, and Z is OH or homoserine residue (including homoserine-lactone residue and symbolized by "Hse"),
or a salt thereof.

2. A stabilized peptide preparation, which comprises a stabilized polypeptide showing motilin-like biological activities and having an amino acid sequence of

```
Phe-Val-Pro-Ile-Phe-Thr-Tyr-Gly-Glu-Leu-Gln-Arg-Leu-Gln-
Glu-Lys-Glu-Arg- Y -Lys-Gly-Gln- Z
```

wherein Y is Gln or Ala, and Z is OH or homoserine residue (including homoserine-lactone residue and symbolized by "Hse"),

3. The use of stabilized peptide according to claim 1 for preparing a stabilized peptide preparation for therapy of gastrointestinal tract disorders.

**Revendications**

1. Polypeptide stabilisé présentant des activités biologiques analogues à celles de la motiline, qui présente la séquence d'acides aminés

```
Phe-Val-Pro-Ile-Phe-Thr-Tyr-Gly-Glu-Leu-Gln-Arg-Leu-Gln-
Glu-Lys-Glu-Arg-Y-Lys-Gly-Gln-Z
```

dans laquelle Y est Gln ou Ala, et Z est OH ou un résidu d'homosérine (y compris un résidu d'homosérine-lactone et symbolisé par "Hse"),
ou un sel de celui-ci.

2. Préparation peptidique stabilisée, qui comprend un polypeptide stabilisé présentant des activités biologiques analogues à celles de la motiline et présentant la séquence d'acides aminés

```
Phe-Val-Pro-Ile-Phe-Thr-Tyr-Gly-Glu-Leu-Gln-Arg-Leu-Gln-
Glu-Lys-Glu-Arg-Y-Lys-Gly-Gln-Z
```

dans laquelle Y est Gln ou Ala, et Z est OH ou un résidu d'homosérine (y compris un résidu d'homosérine-lactone et symbolisé par "Hse").

3. Utilisation du peptide stabilisé selon la revendication 1 pour préparer une préparation peptidique stabilisée pour le traitement des troubles du tract gastrointestinal.

**Patentansprüche**

1. Stabilisiertes Polypeptid, das motilinähnliche biologische Aktivitäten zeigt, das eine Aminosäuresequenz von

**Phe-Val-Pro-Ile-Phe-Thr-Tyr-Gly-Glu-Leu-Gln-Arg-Leu-Gln-Glu-Lys-Glu-Arg- Y -Lys-Gly-Gln- Z,**

worin Y Gln oder Ala darstellt und Z OH oder einen Homoserinrest (einschließlich eines Homoserinlactonrestes und symbolisiert durch "Hse") darstellt, aufweist
oder ein Salz davon.

2. Stabilisierte Peptidzubereitung, umfassend ein stabilisiertes Polypeptid, das motilinähnliche biologische Aktivitäten zeigt und eine Aminosäuresequenz von

**Phe-Val-Pro-Ile-Phe-Thr-Tyr-Gly-Glu-Leu-Gln-Arg-Leu-Gln-Glu-Lys-Glu-Arg- Y -Lys-Gly-Gln- Z,**

worin Y Gln oder Ala darstellt und Z OH oder einen Homoserinrest (einschließlich eines Homoserinlactonrestes und symbolisiert durch "Hse") darstellt, aufweist.

3. Verwendung von stabilisiertem Peptid nach Anspruch 1, zur Herstellung einer stabilisierten Peptidzubereitung zur Therapie von Erkrankungen des Gastrointestinaltrakts.

# FIG. 1

[Leu$^{13}$]motilin−Hse
50.090

A−1
38.136

A−2
41.283

0.00          15.00          30.00          45.00          60.00

Time (min.)

FIG. 2

[Leu¹³] motilin–Hse

Asn(19-position)    Lys(20-position)

[Anhydroaspartic acid residue (19-position)]
[Leu¹³]motilin–Hse

A–1                                              A–2

[Leu¹³, β-Asp¹⁹] motilin–Hse        [Leu¹³, α-Asp¹⁹] motilin–Hse

11